# EUROPÄISCHE PATENTSCHRIFT

(11) **EP 0 946 590 B1**
(45) Veröffentlichungstag und Bekanntmachung des Hinweises auf die Patenterteilung: **17.03.2004**
(21) Anmeldenummer: 97954818.7
(22) Anmeldetag: 17.12.1997
(51) Int. Cl.: C07K 7/08, C07K 1/107, A61K 38/10

(54) **DHC-PEPTID UND DIESES ENTHALTENDES MITTEL**
DIHYDROXYPROPYL CYSTEINE PEPTIDE AND AGENT CONTAINING THIS PEPTIDE
PEPTIDE DU TYPE DIHYDROXYPROPYLCYSTEINE ET AGENT LE CONTENANT

(30) Priorität: 17.12.1996 DE 1965258
(43) Veröffentlichungstag der Anmeldung: 06.10.1999
(73) Patentinhaber: Gesellschaft für Biotechnologische Forschung mbH (GBF), 38124 Braunschweig (DE)
(72) Erfinder: MÜHLRADT, Peter, F., D-38124 Braunschweig (DE)
(74) Vertreter: Boeters, Hans Dietrich, Dr.
(86) Internationale Anmeldenummer: PCT/EP1997/007090
(87) Internationale Veröffentlichungsnummer: WO 1998/027110

(56) Entgegenhaltungen:
- EP-A- 0 519 327
- WO-A-96/16987
- P. MÜHLRADT ET AL.: "Identification of S-(2,3-dihydroxypropyl)cystein in a Macrophage-activating lipopeptide from Mycoplasma fermentans" BIOCHEMISTRY, Bd. 35, 18.Juni 1996, Seiten 7781-7786, XP002070516 in der Anmeldung erwähnt
- R.E. HALL ET AL: "cDNA and genomic cloning and expression of the p48 monocytic differentiation/activation factor, a mycoplasma fermentans gene product" BIOCHEMICAL JOURNAL, Bd. 319, Nr. 3, 1996, Seiten 919-927, XP002070517
- MELCHERS F ET AL: "THE LIPOPROTEIN OF THE OUTER MEMBRANE OF ESCHERICHIA COLI: A B-LYMPHOCYTE MITOGEN" JOURNAL OF EXPERIMENTAL MEDICINE, Bd. 142, Nr. 2, 1.August 1975, Seiten 473-482, XP000574934

## Beschreibung

Die makrophagenstimulierende Wirkung von Mykoplasmen ist seit längerer Zeit bekannt; vgl. Loewenstein et al. in Cellular Immunology, 77 (1983) 290-297. Es ist auch vermutet und formal bewiesen worden, daß Lipoproteine aus Mykoplasmen eine derartige Wirkung zeigen; vgl. Herbelin et al. in Infect. Immun., 62 (1994) 4690-4694 und Mühlradt et al. in Biochemistry, 35 (1996) 7781-7786. Lipoproteine aus Gram-negativen Bakterien und Analoga dieser Lipoproteine sind ebenfalls Immunmodulatoren und speziell als Makrophagenaktivatoren beschrieben worden; vgl. Melchers et al. in J. Exp. Med., 142 (1975) 473-482 und Hoffmann et al. in Immunobiol., 177 (1988) 158-170. Diese Spezies von Lipoproteinen tragen N-terminal eine S-(2,3-Dihydroxypropyl)-cystein-Gruppe (Dhc) mit drei langkettigen Fettsäuren, von denen zwei esterartig und eine amidartig gebunden ist.

Lipoproteine und synthetische Lipopeptidanaloga zeigen eine halbmaximale Wirkkonzentration (Max/2) von etwa 10⁻⁷ M; vgl. Melchers et al. in J. Exp. Med., 142 (1975) 473-482 und Hoffmann et al. in Biol. Chem. Hoppe Seyler, 370 (1989) 575-582.

Synthetische Analoga ohne die Amidfettsäure zeigen eine halbmaximale Wirkkonzentration (Max/2) von etwa 10⁻⁸ M; vgl. Metzger et al. in J. Peptide Scie., 3 (1995) 184-190. Ferner hat Baschang in Tetrahedron, 45 (1989) 6331-6360, 6352 ein mit Taurin modifiziertes Lipopeptid (Na-Sulfonat; CGP-31 362) beschrieben, das nach Dong et al. in J. Exp. Med., 177 (1993) 1071-1077 noch bei 1 bis 10 ng/ml bzw. 1 bis 10 x 10⁻⁹ M makrophagenaktivierend ist. Schließlich beschreiben Metzger et al. in J. Peptide Scie., 3 (1995) 184-190 ein Dhc-Peptid mit der Aminosäuresequenz CFE PPP ATT T, wobei an die 2,3-Dihydroxypropylgruppe zwei Palmitoylgruppen gebunden sind. Die halbmaximale Wirkkonzentration (Max/2) dieses bekannten Peptids beträgt 16 ng/ml bzw. 10 x 10⁻⁹ M.

Es besteht jedoch weiterhin ein Bedürfnis nach wirksamen Lipopeptiden.

Erfindungsgemäß wird nun ein S-(2,3-Dihydroxypropyl)-cysteinpeptid mit zwei esterartig an die Dihydroxypropylgruppe gebundenen Fettsäuren, die gleich oder verschieden sein können, vorgeschlagen, wobei das Peptid die folgende Aminosäuresequenz (I): 
DhcGN NDE SNI SFK EK (I) bzw.
Dhcys Gly Asn Asn Asp Glu Ser Asn Ile Ser Phe Lys
Glu Lys
 oder eine Aminosäuresequenz aufweist, die mit der Sequenz (I) mit der Ausnahme identisch ist, das N-terminal die 2 Aminosäuren an Positionen 2 und gegebenenfalls 3 fehlen und/oder C-terminal 1 bis 2 Aminosäuren deletiert sind.

Erfindungsgemäß können die beiden Fettsäurereste die Formel R-CO- besitzen, wobei R ein C₇₋₂₅-Alkyl-, C₇₋₂₅-Alkenyl- oder C₇₋₂₅-Akinylrest ist, wobei ungesättigte Reste vorzugsweise in cis-Konfiguration vorliegen. Beispiele für C₇₋₂₅-Alkyl-, Alkenyl- und Alkinylreste sind C₁₆- und C₁₈-Reste.

Erfindungsgemäß wird ferner ein Mittel mit einem Gehalt an einem S-(2,3-Dihydroxypropyl)-cystein-peptid gemäß der Erfindung mit einem üblichen Träger und/oder Hilfsmittel vorgesehen. Das erfindungsgemäße Mittel kann zur Stimulierung der Antikörper-Synthese, zur Vorbeugung gegen Infektionen (anti-infective aktivity), als Immunostimulanz gegen Tumoren, zur Aktivierung von Makrophagen, zur Ausbildung von Toleranz gegenüber Endotoxin bzw. bei septischem Schock, insbesondere bei Gram-negativen Bakterien oder als Vakzin-Adjuvanz (Beimischung zu einem Vakzin) verwendet werden.

Erfindungsgemäß lassen sich S-(2,3-Dihydroxypropyl)-cystein-peptide vollsynthetisch herstellen. Der Fachmann kann dabei in Analogie zum angeführten Stand der Technik vorgehen. Verwiesen sei auch auf die DE 35 46 150 A1, DE 37 00 173 A1, DE 38 13 821 A1, DE 41 19 856 A1 und DE 43 29 309 A1.

Nachstehend wird die Erfindung an einem Beispiel näher erläutert.

### Beispiel

Das Lipopeptid wird aus *Mycoplasma fermentans* (beispielsweise PG18) präpariert. Die Isolierung des Lipopeptids aus Mykoplasmen erfolgt durch den folgenden bekannten Trennungsgang (Mühlradt et al. in Biochemistry, 35 (1996) 7781-7786).
(i) Die Delipidierung der Mykoplasmen mit Chloroform/Methanol.
(ii) Extraktion der delipidierten Mykoplasmen mit heißem 25 mM Octylglucosid.
(iii) Dialyse dieses Detergens-Extraktes.
(iv) Konzentrieren des Extraktes durch Gefriertrocknung.
(v) Umkehrphasen-Chromatographie auf einer C8-Säule mit einem Wasser/2-Propanol-Gradienten.
Die Detektion der biologischen Aktivität erfolgt, indem man Nitrit und Nitrat als Folgeprodukte von Stickstoffmonoxid mißt, das bei Stimulation von interferonbehandelten murinen Peritonealmakrophagen freigesetzt wird.

Der Wirkstoff ist ein S-(2,3-Dihydroxypropyl)-cystein-peptid mit zwei esterartig gebundenen langkettigen Fettsäuren (C16:0 und C18:0/C18:1) an der Dihydroxypropylgruppe und mit der folgenden Sequenz:
Dhc-GNN DES NIS FKE K. Das häufigste Molekulargewicht beträgt 2164. Daneben können Varianten ermittelt werden, die sich durch unterschiedliche Fettsäuren und durch ein um zwei Aminosäuren C-terminal verkürztes Peptid auszeichnen.

Die Substanz besitzt die Eigenschaft, Makrophagen von Mäusen und Menschen zur Freisetzung von Zytokinen und Prostaglandinen zu stimulieren, und zwar mit allen Konsequenzen der indirekten Stimulation von T- und B-Lymphozyten; vgl. Mühlradt et al. in Infect. Immun., 59 (1991) 3962-3968 und Feng & Lo in Infect. Immun., 62 (1994) 3916-3921. Ihre halbmaximale Wirkkonzentration (Max/2) beträgt 20 pg/ml bzw. 10⁻¹¹ M im Maussystem. Diese Wirkkonzentration ist um den Faktor 10² bis 10³ geringer als die entsprechenden bekannten Konzentrationen-ähnlicher natürlicher oder synthetischer Lipopeptide.

## Patentansprüche

1. S-(2,3-Dihydroxypropyl)-cystein-peptid mit zwei esterartig an die Dihydroxypropylgruppe gebundenen Fettsäuren, die gleich oder verschieden sein können, wobei das Peptid die folgende Aminosäuresequenz (I): DhcGN NDE SNI SFK EK oder eine Aminosäuresequenz aufweist, die mit der Sequenz (I) mit der Ausnahme identisch ist, daß N-terminal die Aminosäuren an Positionen 2 und gegebenenfalls 3 fehlen und/oder C-terminal 1 bis 2 Aminosäuren deletiert sind.

2. S-(2,3-Dihydroxypropyl)-cystein-peptid nach Anspruch 1, **dadurch *gekennzeichnet*, daß** die beiden Fettsäurereste die Formel R-CO- besitzen, wobei R eine C₇₋₂₅-Alkyl-, C₇₋₂₅-Alkenyl- oder C₇₋₂₅-Alkinylgruppe ist, wobei ungesättigte Reste vorzugsweise in cis-Konfiguration vorliegen.

3. Mittel mit einem Gehalt an einem S-(2,3-Dihydroxypropyl)-cystein-peptid gemäß Anspruch 1 oder 2 neben einem üblichen Träger und/oder Hilfsmittel.

4. Mittel nach Anspruch 3 zur Stimulierung der Antikörper-Synthese.

5. Mittel nach Anspruch 3 zur Vorbeugung gegen Infektionen (anti-infective activity).

6. Mittel nach Anspruch 3 zur Immunostimulanz gegen Tumoren.

7. Mittel nach Anspruch 3 zur Aktivierung von Makrophagen.

8. Mittel nach Anspruch 3 zur Ausbildung von Toleranz gegenüber Endotoxinen (septischer Schock), insbesondere bei Gram-negativen Bakterien.

9. Mittel nach Anspruch 3 als Vakzin-Adjuvanz (Beimischung zu einem Vakzin).

10. Mittel nach Anspruch 3 für kurzfristigen Schutz vor Infektionen, insbesondere Crowding Disease, bei Tieren, insbesondere Vieh, Nutz- oder Haustieren.

## Claims

1. S-(2,3-Dihydroxypropyl)-cysteine peptide having two fatty acids, which may be identical or different, bonded in the form of esters to the dihydroxypropyl group, the peptide having the following amino acid sequence (I): DhcGN NDE SNI SFK EK or an amino acid sequence that is identical to sequence (I) except that the N-terminal amino acids in positions 2 and, optionally, 3 are missing and/or one or two C-terminal amino acids have been deleted.

2. S-(2,3-Dihydroxypropyl)-cysteine peptide according to claim 1, **characterized in that** the two fatty acids radicals have the formula R-CO-, wherein R is a C₇-C₂₅-alkyl, C₇-C₂₅-alkenyl or C₇-C₂₅-alkynyl group, unsaturated radicals preferably being present in the cis configuration.

3. Composition comprising a S-(2,3-Dihydroxypropyl)-cysteine peptide according to claim 1 or 2 together with a conventional carrier and/or adjuvant.

4. Composition according to claim 3 for stimulating the synthesis of antibodies.

5. Composition according to claim 3 for preventing infections (anti-infective activity).

6. Composition according to claim 3 for immunostimulation against tumours.

7. Composition according to claim 3 for activating macrophages.

8. Composition according to claim 3 for developing tolerance towards endotoxins (septic shock), especially in the case of Gram-negative bacteria.

9. Composition according to claim 3 as a vaccine adjuvant (admixture with a vaccine).

10. Composition according to claim 3 for short-term protection against infections, especially Crowding disease, in animals, especially livestock, working animals or domestic animals.

## Revendications

1. Peptide à S-(2,3-dihydroxypropyl)-cystéine, portant deux restes d'acide gras, identiques ou différents, liés en fonctions esters au groupe dihydroxypropyle, ledit peptide présentant la séquence suivante d'acides aminés : DhcGN NDE SNI SFK EK (I) ou une séquence d'acides aminés qui est identique à la séquence (I), à ceci près que du côté N-terminal, l'acide aminé placé en position 2, et le cas échéant, celui placé en position 3 fait ou font défaut, et/ou qu'un à deux acides aminés de l'extrémité C-terminale sont enlevés.

2. Peptide à S-(2,3-dihydroxypropyl)-cystéine, conforme à la revendication 1, **caractérisé en ce que** les deux restes d'acide gras ont pour formule R-CO- où R représente un groupe alkyle en C₇₋₂₅, alcényle en C₇₋₂₅ ou alcynyle en C₇₋₂₅, les restes insaturés se trouvant de préférence dans la con-figuration cis.

3. Agent contenant un peptide à S-(2,3-dihydroxypropyl)-cystéine, conforme à la revendication 1 ou 2, ainsi qu'un véhicule et/ou un adjuvant habituels.

4. Agent conforme à la revendication 3, pour stimulation de la synthèse d'anticorps.

5. Agent conforme à la revendication 3, pour prévention contre les infections (activité anti-infectieuse).

6. Agent conforme à la revendication 3, pour immunostimulation contre les tumeurs.

7. Agent conforme à la revendication 3, pour activation des macrophages.

8. Agent conforme à la revendication 3, pour constitution d'une tolérance vis-à-vis d'endotoxines (choc septique), en particulier dans le cas de bactéries Gram-négatives.

9. Agent conforme à la revendication 3, comme adjuvant de vaccin (à mélanger à un vaccin).

10. Agent conforme à la revendication 3, pour protection à court terme contre des infections, en particulier la maladie de Crowding, chez des animaux, en particulier le bétail, les animaux domestiques et les animaux familiers.
